# EUROPÄISCHE PATENTSCHRIFT

(11) **EP 1 567 510 B1**
(45) Veröffentlichungstag und Bekanntmachung des Hinweises auf die Patenterteilung: **20.12.2006**
(21) Anmeldenummer: 03812092.9
(22) Anmeldetag: 31.03.2003
(51) Int. Cl.: C07D 295/10

(54) **VERFAHREN ZUM HERSTELLEN VON SALZEN DES TOLPERISON**
METHOD FOR PRODUCING SALTS OF TOLPERISONE
PROCEDE DE PRODUCTION DE SELS DE TOLPERISONE

(30) Priorität: 05.12.2002 AT 18232002
(43) Veröffentlichungstag der Anmeldung: 31.08.2005
(73) Patentinhaber: Sanochemia Pharmazeutika AG, 1090 Wien (AT)
(72) Erfinder: CZOLLNER, Laszlo, A-2490 Ebenfurth (AT); KÄLZ, Beate, A-7035 Steinbrunn (AT); ROTHENBURGER, Jan, A-2604 Theresienfeld (AT); WELZIG, Stefan, A-1030 Wien (AT)
(74) Vertreter: Hehenberger, Reinhard
(86) Internationale Anmeldenummer: PCT/AT2003/000092
(87) Internationale Veröffentlichungsnummer: WO 2004/050648

(56) Entgegenhaltungen:
- SUMITA K ET AL: "A MODIFIED MANNICH REACTION USING 1,3-DIOXOLANE" CHEMICAL AND PHARMACEUTICAL BULLETIN, PHARMACEUTICAL SOCIETY OF JAPAN. TOKYO, JP, Bd. 42, Nr. 8, 1. August 1994 (1994-08-01), Seiten 1676-1678, XP002059561 ISSN: 0009-2363 in der Anmeldung erwähnt
- DATABASE CAPLUS [Online] CHEMICAL ABSTRACTS SERVICE, COLUMBUS, OHIO, US; YOKOYAMA, YUKIO ET AL: "4'-Substituted-2-methyl-3-piperidinopropi ophenones" retrieved from STN Database accession no. 88:22646 XP002252004 -& JP 52 095674 A (NIPPON CARBIDE INDUSTRIES CO., INC., JAPAN) 11. August 1977 (1977-08-11)

## Beschreibung

Die Erfindung betrifft ein Verfahren zum Herstellen von Salzen und Hydraten von Tolperison durch Addition organischer und anorganischer Säuren mit der allgemeinen Formel B:

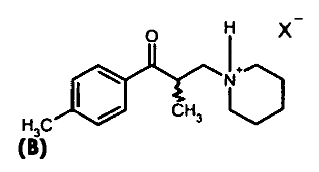

Tolperison ist der internationale Freiname für das Muskelrelaxans (RS)-2,4'-Dimethyl-3-piperidinopropiophenon mit der Summenformel C₁₆H₂₃NO. Tolperison ist ein muskelentspannendes Mittel (Muskelrelaxans), das an Bedeutung gewinnt, mit der nachstehenden Formel A

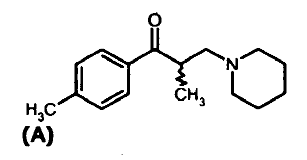

Hauptindikationen von Tolperison sind Erkrankungen, die mit schmerzhaften Muskelverspannungen einhergehen, z.B. Wirbelsäulensyndrome, muskuläre Schmerzen bei degenerativen Erkrankungen, berufs- und sportbedingte Überlastungssyndrome und das Fibromyalgiesyndrom.

Ein Vorteil der Behandlung mit Tolperison ist, dass auch funktionelle Parameter, wie z.B. die Mobilität des Patienten, verbessert werden. Patienten mit langfristiger Einnahme von Tolperison haben durch das Fehlen zentraler Nebenwirkungen in der Regel ein gutes therapeutisches Verhältnis und die zum Therapieerfolg notwendige Vertrauensbasis zum weiteren Einsatz dieses Medikaments.

Tolperison und dessen Salze mit den allgemeinen Formeln A und B sind bekannt und können auf verschiedenen chemischen Wegen hergestellt werden. Die bekannten Verfahren haben den Nachteil, dass sie auf Grund niedriger Ausbeuten, oder kommerziell nicht erhältlicher Ausgangsmaterialien, sowie auf Grund komplizierter Reaktionsbedingungen im Industriemaßstab nur schwer durchführbar sind.

### J. Labelled Cpd. Radiopharm 42, 1125-1134 (1999)

Um radioaktiv markiertes Tolperison herstellen zu können, schlugen Ditriech und Fels einen Syntheseweg ausgehend von 4'-Methylacetophenon und Paraformaldehyd vor. Die mehrstufige Synthese führt zu einem Substanzgemisch und Tolperison kann nur durch Säulenchromatographie isoliert werden.

### Jap. Pat.4005283 A2 19920109

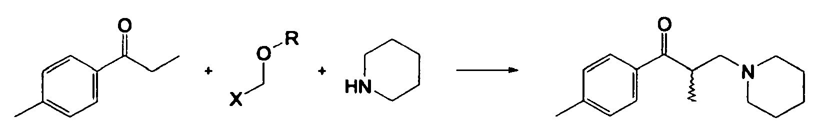

Nachteile dieses Verfahrens sind die mehrstufige Synthese, das Zwischenprodukt muß isoliert und gereinigt werden.

### Jap. Pat. 54032480 19790309

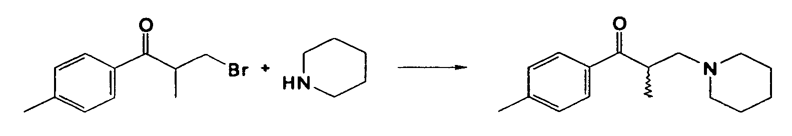

Die Herstellung des Halogen-Derivates ist aufwändig und teuer.

### Jap. Pat. 54036274 19790316

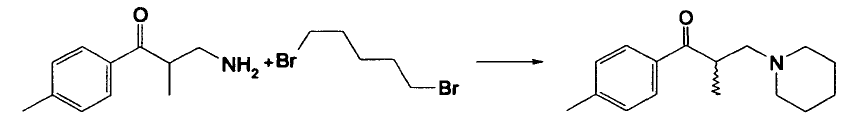

Dieser Syntheseweg geht von teuren Ausgangsmaterialien aus und es entstehen mehreren Nebenprodukte (wg. Dibrompentan).

### Jap. Pat. 54030178 19790306

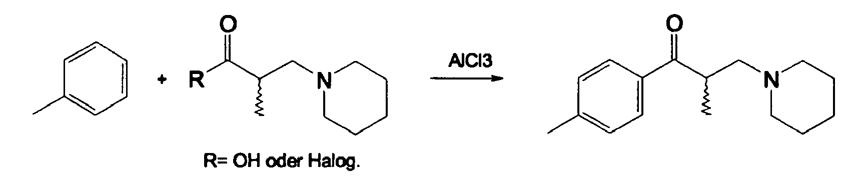

Das zu diesem Syntheseweg benötigte Ausgangsmaterial muss über mehrere Stufen hergestellt werden. Desweiteren muss unter Feuchtigkeitsausschluss gearbeitet werden um die Hydrolyse von Aluminiumtrichlorid zu vermeiden.

### Jap. Pat. 54027571 19790301

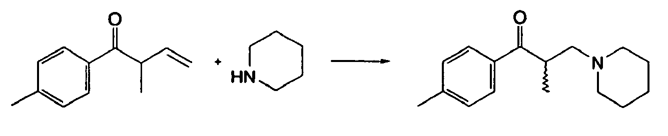

Dieser Syntheseweg geht von teuren Ausgangsmaterialien aus und es entstehen mehreren Nebenprodukte (wg. Vinylverbindung).

### Chem. Pharm. Bull. 42,1676 (1994):

Kazuharu et al. beschrieben in Chem. Pharm. Bull 42(8) 1676 (1994) die Herstellung von Tolperison durch Mannich-Reaktion. Die publizierten Ausbeuten sind zwar relativ hoch, durch die wässrige Extraktion bilden sich allerdings mehrere Nebenprodukte. Nachteilig und teuer ist die mehrstufige Aufarbeitung, da die Substanz zunächst in öliger Form und erst anschließend als Hydrochlorid isoliert wird.

### RO 75-83082 19750804 (CAN 98: 125629) RO-A-68297

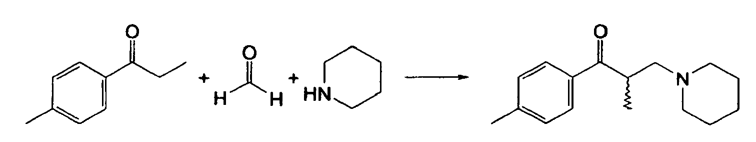

Die Anwendung von Formaldehyd in nicht geschützter Form hat mehrere Nachteile, wie Wasser in Reaktionsgemisch, hohe Toxizität (IHL-TCLO HMN 17 mg/m³/30m; ORL-RAT LD50 100 mg kg⁻¹) oder Bildung von sehr schwer löslichem Paraformaldehyd.

### Jap. Pat. 20,390 (1965)

Matatsugu et al. veröffentlichten eine Methode zur Herstellung von Tolperison ausgehend von Paraformaldehyd in einer Mischung aus Nitromethan : Ethanol : Toluol (40:5,5:11) unter Verwendung von wässriger Salzsäure. Die angegebene Reaktionsführung ergibt ein Konglomerat und das Arbeiten mit Nitromethan ist aufgrund seiner Gefährlichkeit teuer.

### Hung. Pat. 144,997 (1956)

Nádor et al. beschreiben ein großtechnisches Verfahren zur Herstellung von Tolperison unter Verwendung von mit gasförmigem Formaldehyd gesättigten Ethanol. Dieses Verfahren führt zwar sofort zu Tolperison Hydrochlorid, die Ausbeuten sind aber niedrig und das Arbeiten mit gasförmigem Formaldehyd ist aufgrund seiner Gefährlichkeit teuer.

Unter unerwünschten Isomeren sind insbesondere gemeint:
(C): 2-Methyl-1-(3-methylphenyl)-3-piperidin-1-ylpropan-1-on;
(D): 2-Methyl-1-(2-methylphenyl)-3-piperidin-1-ylpropan-1-on;

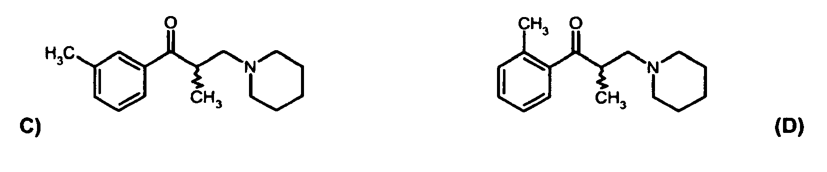

Ausgehend von der Synthesemethode und der Qualität (Reinheit) der eingesetzten Ausgangsstoffe sind bisher folgende Verunreinigungen des Endproduktes (Tolperison) möglich:

| **Bezeichnung** | ***Chemischer Name*** | ***Chemische Struktur*** |
|---|---|---|
| Piperidine HCl | piperidine hydrochloride | 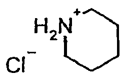 |
| C | 2-methyl-1-(3-methylphenyl)-3-(1-piperidinyl)-propanone hydrochloride | 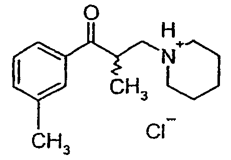 |
| | 3-tolperisone hydrochloride | |
| 4-MPP | 1-(4-methylphenyl)-propanone | 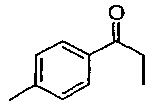 |
| | 4-methylpropiophenone | |
| E | 2-methyl-1-(4-methylphenyl)-propenone | 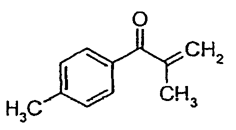 |
| D | 2-methyl-1-(2-methylphenyl)-3-(1-piperidinyl)-propanpone hydrochloride | 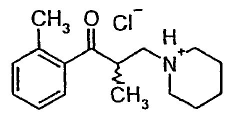 |
| | 2-tolperisone hydrochloride | |

Der Erfindung liegt die Aufgabe zugrunde, ein verbessertes, in technischem Maßstab durchführbares Verfahren zum Herstellen von Tolperison mit verbesserter Reinheit und dessen Salzen zu entwickeln, ohne dass die Nachteile der bekannten Synthesewege auftreten.

Gelöst wird diese Aufgabe mit einem Verfahren, das die Merkmale von Patentanspruch 1 aufweist.

Vorteilhafte Ausgestaltungen des erfindungsgemäßen Verfahren sind Gegenstand der Unteransprüche.

Mit den nachstehend beschriebenen analytischen Methoden können diese Verunreinigungen bis in einen Grenzbereich von 0,1 % nachgewiesen werden.

### 1.1. Methode 1: Prüfung Gehalt Tolperison und Verunreinigungen 3-Tolpersion (C), 4-Methylpropiophenon (4-MPP) und Vinylketon (E):

Die Bestimmung des Gehaltes von Tolperison und den genannten Verunreinigungen erfolgt mittels Messung gegen externe Standards auf einem HPLC System mit UV-Detektion. Die stationäre Phase besteht aus einem funktionafisierten Polysaccharid. Als mobile Phase wird ein binäres System aus Borat-Puffer und einem organischen Modifier (Acetonitril) verwendet.

### 1.2. Methode 2: Prüfung Gehalt 2-Tolperison (D):

Zur Bestimmung des Gehaltes von 2- Tolperison wird ebenfalls ein HPLC System mit UV-Detektion verwendet. Die stationäre Phase ist ein auf Silikat gebundenes Calixaren. Als mobile Phase wird eine Mischung aus Phosphat - Puffer und Methanol verwendet.

### 1.3. Methode 3: Prüfung Gehalt Piperidine Hydrochlorid.

Zur Bestimmung von Piperidin HCl wird eine quantitative LC/MS Methode verwendet. Die stationäre Phase besteht aus octadecysilyliertem Kieselgel. Die binären mobile Phase enthält Trichloressigsäure und Methanol.

Mit den beschriebenen Methoden können auch die Verbindungen C und D nachgewiesen (bestimmt) werden, obwohl sich diese Stellungsisomere in ihren chemischen Eigenschaften kaum von Tolperison unterscheiden und daher schwer voneinander zu trennen sind.

Bisher wurde zur Gehaltsbestimmung von Tolperison eine titrimetische Methode verwendet (Pharm. Jap. XI), mit der nur die Summe der Verbindungen B, C und D erfasst werden kann.

Durch die neue HPLC-Methode zum Bestimmen von Tolperison, 2-Tolperison, 3-Tolperison und den übrigen Verunreinigungen und der LC/MS-Methode zum Bestimmen von Piperidin Hydrochlorid, sind die Probleme der bekannten Analysen beseitigt.

Mit diesen Analysenverfahren wurde die Reinheit von erfindungsgemäß hergestelltem Tolperison und von Tolperison in auf dem Markt befindlichen Präparaten bestimmt.

Die Ergebnisse sind in der nachstehenden Tabelle zusammengefasst:

| **Charge** | **2-Tolperison** | **3-Tolperison** | **Piperidin** | **Vinylketon** | **4-MPP** |
|---|---|---|---|---|---|
| Mydeton 50 mg Tabl.von Gedeon Richter | 0.3 % | 0.8 % | < 0.05 % | < 0.05 % | < 0.05 % |
| Mydeton 150 mg Tabl. von Gedeon Richter | 0.6% | 1.2% | <0.05% | <0.05% | < 0.05 % |
| Mydocalm 50 mg Tabl von Strathmann | 0.6 % | 1.1% | <0.05% | <0.05% | <0.05 % |
| Tolperison Erfindung | < 0.05 % | 0.1 % | <0.05 % | < 0.05 % | < 0.05 % |

Die in der Tabelle zusammengefassten Analysen zeigen, dass Tolperison in auf dem Markt befindlichen Produkte in seinem Reinheitsprofil, besonders im Gehalt an Stellungsisomeren, deutlich schlechter ist, als das gemäß der vorliegenden Erfindung erhältliche Tolperison und damit nicht den aktuellen Richtlinien der europäischen Behörden entsprechen.

Das erfindungsgemäße Verfahren zur Synthese von Tolperison lässt sich wie folgt wiedergeben:

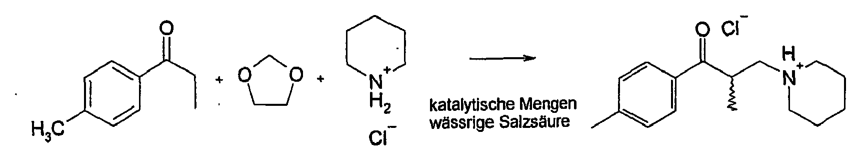

Als Ausgangsmeterial werden 4-Methylpropiophenon, Piperidin Hydrochlorid und 1,3-Dioxolon als Reaktionspartner und letzteres in einer bevorzugten Ausführungsform auch als Lösungsmittel eingesetzt.
Das Verwenden von 1,3-Dioxolan an Stelle von Formaldehyd und die hohe Ausbeute nach der direkten Isolierung von Tolperison machen die einstufige Reaktion auch im Industriemaßstab wirtschaftlich.

Vorteilhaft bei dem erfindungsgemäßen Verfahren ist, dass das eingesetzte 4-Methylpropiophenon mit bis zu 5 % 3-Methylpropiophenon und bis zu 2 % 2-Methylpropiophenon verunreinigt sein darf, und mit dem erfindungsgemäßen Verfahren dennoch die nötige Endproduktreinheit erlangt wird.

Bei dem erfindungsgemäßen Verfahren zum Herstellen von Salzen von Tolperison der Formel **B** kann in einer Ausführungsform für die in 1,3-Dioxolan durchgeführte Aminomethylierung wässerige Salzsäure in katalytischen Mengen eingesetzt werden. Dadurch kann das Endprodukt durch Zugabe von Ethylacetat und tert.-Buthyl-methylether als leicht abtrennbares Salz des Tolperisons, gemäß allgemeiner Formel **B** beispielsweise als Chlorid (X = Cl) hergestellt und aus dem Reaktionsgemisch durch Fällung abgetrennt werden kann.

Das abgetrennte Salz von Tolperison besitzt bereits hohe Reinheit und ein günstiges Verunreinigungsprofil, kann jedoch bei Bedarf z.B. durch weiteres Umkristallisieren weiter gereinigt werden.

Insgesamt ist das Verfahren der Erfindung auch zur Durchführung im Industriemaßstab geeignet, da ein Reinigungsschritt mittels Salzfällung auch in industriellem Maßstab nur einen geringen Aufwand darstellt. Das Verfahren der Erfindung lässt sich auch automatisiert ausführen.

Das Verfahren der Erfindung erlaubt das Herstellen von Salzen von Tolperison durch Addition von für die Pharmazeutik geeigneter Säuren, Bevorzugte Säuren sind Mineralsäuren, und hier insbesondere die Salzsäure.

Vorteilhafte erfindungsgemäße Verfahrensvarianten werden anhand der folgenden, Beispiele näher erläutert:

### Beispiel 1:

### Herstellung von Tolperison-Hydrochlorid

In einem 3-L-Dreihalskolben mit Rückflußkühler, Kalziumchloridrohr, Argoneinleitrohr und Rührer werden 200 ml 1,3-Dioxolan und 146,2 ml 4-Methylpropiophenon vorgelegt und mit 100 g Piperidin Hydrochlorid und 4,0 ml 33%iger wässeriger Salzsäure versetzt. Der Pulvertrichter wird mit 20 ml 1,3-Dioxolan nachgewaschen, der Rührer eingeschaltet und das Reaktionsgefäß verschlossen. Die Reaktionsmischung wird einmal mit Argon gespühlt und bei 100 - 105°C Badtemperatur (83 - 86°C Innentemperatur) gerührt. Der weiße Niederschlag löst sich nach etwa 15-16 Stunden auf. Nach 18-20 Stunden zeigt das Dünnschichtchromatogramm kein Piperidin mehr. Nach 24 Stunden wird die Heizung ausgeschaltet, das Ölbad entfernt, unter heftigen Rühren die klare Reaktionslösung noch warm mit 800 ml Ethylacetat versetzt, auf Raumtemperatur gekühlt und mit 400 ml Methyl-tert.-Butylether (MTBE) versetzt. Der ausgefallene Niederschlag wird bei 0 bis 10 °C weitere 2 Stunden gerührt, über eine Glasnutsche PO-3 abfiltriert und der Niederschlag zweimal mit je 200 ml MTBE nachgewaschen. Die Substanz wird im Vakuumtrockenschrank bei 75 - 80°C und 20 - 40mbar 16 bis 24 Stunden getrocknet.
Ausbeute: 206,5 g (89,1 %, berechnet auf Piperidin Hydrochlorid) farbloses Pulver Smp.: 169 °C

| Schmelzpunkt | 2-Tolperison | 3-Tolperison | 4-Tolperison | Piperidin | Verunr. E | 4-MPP |
|---|---|---|---|---|---|---|
| 169°C | 0.22 % | 0.30 % | 98.0 % | < 0.05 % | < 0.05 % | < 0.05 % |

### Beispiel 2: Reinigung von Tolperison-Hydrochlorid

In einen 500ml-Dreihalskolben mit Rührer, Rückflußkühler und Tropftrichter werden 58,0 g Tolperison vorgelegt und mit 87 ml Isopropylalkohol versetzt. Die Reaktionsmischung wird bis zum Siedepunkt erhitzt, wobei eine klare Lösung entsteht. Die warme Reaktionslösung wird mit 2b1 ml MTBE versetzt und unter ständigem Rühren auf Raumtemperatur gekühlt. Die entstandene Suspension wird bei Raumtemperatur 14 - 18 Stunden gerührt, auf 5 - 10°C gekühlt und nach 2-3 Stunden Rühren abfiltriert. Der Niederschlag wird zweimal mit je 80 ml MTBE nachgewaschen und im Vakuumtrockenschrank bei 55 - 60°C und 30 - 50mbar 14 bis 24 Stunden getrocknet.
Ausbeute: 48,0 g (82,9 %) farblose Substanz
Smp.: 171 °C
Analyse:

| Schmelzpunkt | 2-Tolperison | 3-Tolperison | 4-Tolperison | Piperidin | Verunr. E | 4-MPP |
|---|---|---|---|---|---|---|
| 171°C | <0.05% | 0.16% | 98.9% | <0.05% | <0.05% | <0.05% |

### Beispiel 3:

### Industrielle Herstellung von Tolperison-Hydrochlorid

75 kg Piperidin Hydrochlorid und 105 kg 4-Methylpropiophenon werden in 180 kg 1,3-Dioxolan und 12 kg Salzsäure bei 90°C unter Stickstoffatmosphäre für 7 bis 20 Stunden erhitzt. Durch Zugabe von 500 kg Ethylacetat und 440 kg MTBE bei erhöhter Temperatur (40 - 80°C) wird eine Suspension des Produktes erhalten. Nach der Fest - Flüssig - Trennung wird das feuchte Produkt bei 60 - 80°C im Vakuum (200 - 500 mbar) über 12 - 24 Stunden getroknet, wodurch 140 kg (81,5 %) farblose Kristalle erhalten werden.
Smp: 170°C
Analyse:

| Schmelzpunkt | 2-Tolperison | 3-Tolperison | 4-Tolperison | Piperidin | Verunr. E | 4-MPP |
|---|---|---|---|---|---|---|
| 170°C | 0.47 | 0.36 | 97.8 | 0.9 % | < 0.05 % | < 0.05 % |

### Beispiel 4: Großtechnisches Umkristallisieren von Tolperison-Hydrochlorid

60 kg Tolperison (aus Beispiel 3) werden in 410 kg 2-Butanon und 71 kg Isopropanol unter Stickstoffatmosphäre auf Rückfluß erhitzt. Nach einer optionalen Heißfiltration wird durch Abkühlen eine Suspension des Produktes erhalten. Nach der Fest - Flüssig - Trennung wird das feuchte Produkt bei 60 - 80°C im Vakuum (200 - 500 mbar) über 12-24 Stunden getrocknet, wodurch 45 kg (75%) farblose Kristalle erhalten werden.
Smp.: 173°C
Analyse:

| Schmelzpunkt | 2-Tolperison | 3-Tolperison | 4-Tolperison | Piperidin | Verunr. E | 4-MPP |
|---|---|---|---|---|---|---|
| 173°C | <0.05% | 0.14% | 98.5% | < 0.05 % | < 0.05 % | <0.05 % |

### Beispiel 5: Industrielle Herstellung von Tolperison-Hydrochlorid

107 kg Piperidin-Hydrochlorid und 150 kg 4-Methylpropionphenon werden in 159 kg 1,3-Dioxolan und 107 L Salzsäure bei 90°C unter Stickstoffatmosphäre für 7 bis 20 Stunden erhitzt. Durch Zugabe von 783 kg Ethylacetat und 322 kg Methyl-tert.-Butylether bei erhöhter Temperatur (40-80°C) wird eine Suspension des Produktes erhalten. Nach der Fest-Flüssig-Trennung wird das feuchte Produkt bei 60-80°C im Vakuum (200-500 mbar) über 12-24 Stunden getrocknet, wodurch 200 kg (81,5%) farblose Kristalle erhalten werden.
Smp.: 170°C

### Beispiel 6: Großtechnisches Umkristallisieren von Tolperison-Hydrochlorid

190 kg Tolperison (aus Beispiel 5) werden in 1300 kg 2-Butanon und 224 kg Isopropanol unter Stickstoffatmosphäre auf Rückfluß erhitzt. Nach einer optimalen Heißfiltration wird durch Abkühlen eine Suspension des Produktes erhalten. Nach der Fest-Flüssig-Trennung wird das feuchte Produkt bei 60-80°C im Vakuum (200-500 mbar) über 12-24 Stunden getrocknet, wodurch 143 kg (75%) farblose Kristalle erhalten werden.
Smp.: 173°C

Zusammenfassend kann ein Ausführungsbeispiel der Erfindung wie folgt dargestellt werden:

Beschrieben wird ein Verfahren zum Herstellen von einem Additionssalz von 2,4'-Dimethyl-3-piperidinopropiophenon (Tolperison) mit einer pharmazeutisch annehmbaren Säure, der Formel 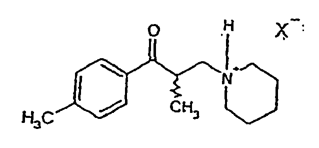 bei dem 4-Methylpropiophenon mit Piperidin-Hydrochlorid und 1,3-Dioxolan in Gegenwart einer Säure als Katalysator umgesetzt wird und das erhaltene Tolperison als Säureadditionssalz nach dem Abkühlen der Reaktionsmischung aus derselben durch Zugabe von Ethylacetat und tert.-Butylmethylether durch Fällung abgetrennt wird.

## Patentansprüche

1. Verfahren zum Herstellen von einem Additionssatz von 2,4'-Dimethyl-3-piperidinopropiophenon (Tolperison), der Formel (A) 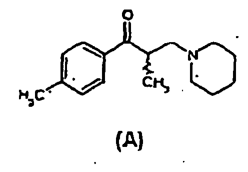 mit einer pharmazeutisch annehmbaren Säure, der Formel (B) 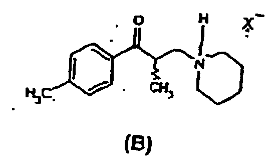 wobei man 4-Methylpropiophenon der Formel 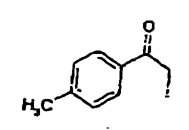 mit Piperidin-Hydrochlorid der Formel 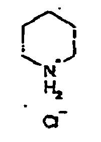 und 1,3-Dioxolan der Formel 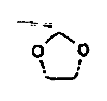 in Gegenwart einer Säure ais Katalysator umsetzt, **dadurch gekennzeichnet, dass** Tolperison als Sövreadditionssalz gemäß der allgemeinen Formel (B) nach dem Abkühlen der Reaktionsmischung aus derselben durch Zugabe von Ethylacetat und tert-Butylmethylether durch Fällung abgetrennt wird.

2. Verfahren noch Anspruch 1, **dadurch gekennzeichnet, daß** die Reaktion in Gegenwart katalytischer Mengen einer Säure, insbesondere wässeriger Salzsäure, ausgeführt wird.

3. Verfahren nach Anspruch 1 oder 2, **dadurch gekennzeichnet, daß** die Reaktion in einem Lösungsmittel ausgeführt wird.

4. Verfahren nach Anspruch 3, **dadurch gekennzeichnet, daß** die Reaktion in 1,3-Dioxolan als Lösungsmittel in einem Konzentrationsbereich von 1 bis 6. vorzugsweise 3,6 mol/lit. durchgeführt wird.

5. Verfahren nach einem der Ansprüche 1 bis 4. **dadurch gekennzeichnet, daß** dos erhaltene Tolperison mit einer anorganischen Säure, wie Chlorwassentoffsäure, in das Additionssalz übergeführt wird.

## Claims

1. Method for manufacturing an addition salt of 2,4'-dimethyl-3-piperidinopropiophenone (tolperisone), of formula (A) 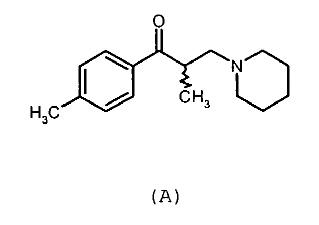 with a pharmaceutically acceptable acid, of formula (B) 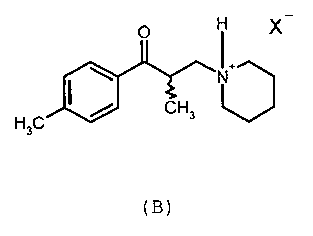 whereby 4-methylpropiophenone of the formula 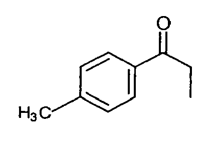 is reacted with piperidine hydrochloride of formula 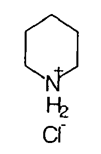 and 1,3-dioxolane of the formula 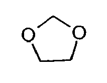 in presence of an acid as catalyst, **characterized by** the fact that tolperisone is separated as an acid addition salt in accordance with the general formula (B) after cooling the reaction mixture by addition of ethyl acetate and tert-butylmethylether to cause precipitation.

2. Method according to claim 1, **characterized by** the fact that the reaction is carried out in the presence of catalytic quantities of an acid, in particular aqueous hydrochlorid acid.

3. Method according to claim 1 or 2, **characterized by** the fact that the reaction is carried out in a solvent.

4. Method according to claim 3, **characterized by** the fact that the reaction is accomplished in 1,3-dioxolane as a solvent in a concentration range from 1 to 6, preferably 3,6 mol/lit.

5. Method according to one of claims 1 to 4, **characterized by** the fact that the resulting tolperisone with the inorganic acid, such as hydrochloric acid, is converted into the addition salt.

## Revendications

1. Procédé de production d'un sel d'addition de 2,4'-diméthyl-3-pipéridinopropiophénone (tolpérisone) de formule (A) 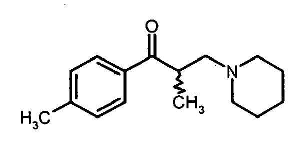 avec un acide pharmaceutiquement acceptable de formule (B) 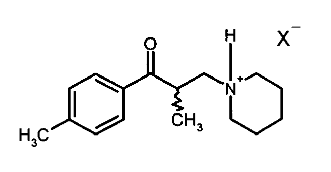 dans lequel on fait réagir le 4-méthylpropiophénone de formule 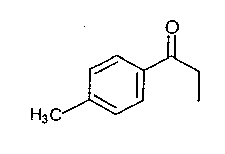 avec un hydrochlorure de pipéridine de formule 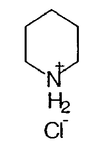 et le 1,3-dioxolane de formule 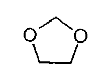 en présence d'un acide comme catalyseur, **caractérisé en ce que** la tolpérisone en tant que sel d'addition acide selon la formule générale (B) est séparée par précipitation après refroidissement du mélange réactionnel de ceux-ci en ajoutant de l'acétate d'éthyle et du tert.-butylméthyléther.

2. Procédé selon la revendication 1, **caractérisé en ce que** la réaction est conduite en présence de quantités catalytiques d'un acide, en particulier d'acide chlorhydrique aqueux.

3. Procédé selon la revendication 1 ou 2, **caractérisé en ce que** la réaction est conduite dans un solvant.

4. Procédé selon la revendication 3, **caractérisé en ce que** la réaction est conduite dans du 1,3-dioxolane comme solvant dans une plage de concentrations de 1 à 6, de préférence 3,6 moles/litre.

5. Procédé selon l'une quelconque des revendications 1 à 4, **caractérisé en ce que** la tolpérisone obtenue est transformée en sel d'addition avec un acide anorganique tel que l'acide chlorhydrique.
